# EUROPEAN PATENT APPLICATION

(11) **EP 1 847 255 A2**
(43) Date of publication of application: **24.10.2007**
(21) Application number: 07014139.5
(22) Date of filing: 27.12.2001
(51) Int. Cl.: A61K 9/00

(54) **Sustained release drug delivery devices with coated drug cores**

(30) Priority: 03.01.2001 US 259251 P
(62) Divisional of application: 01991450.6
(71) Applicant: BAUSCH & LOMB INCORPORATED, Rochester, NY 14604-2701 (US)
(72) Inventor: Viscasillas, Santos, Tampa, Florida 33647 (US)
(74) Representative: Bowman, Paul Alan

(57) **Abstract**

A sustained release drug delivery device comprising:
a) a drug core comprising an inner core comprising at least one agent effective in obtaining a diagnostic effect or effective in obtaining a desired local or systemic physiological or pharmacological effect and a polymer coating layer, the polymer being permeable to the passage of said agent, wherein the polymer coating layer covers the inner core; and
b) a reservoir essentially impermeable to the passage of said agent that surrounds and defines an internal compartment to accept said drug core, said reservoir comprising at least one passageway, said passageway allowing passage of said agent out of the inner core, through the polymer coating layer, through the passageway, and out of the device.

## Description

### FIELD OF THE INVENTION

The present invention relates to an improved device and method for delivering drug directly to the interior portions of the body of a mammalian organism, such as to the eye. The method includes administration of an agent effective in obtaining a desired diagnostic effect or local or systemic physiological or pharmacological effect by inserting in a desired location in the body of a mammalian organism a sustained release drug delivery device.

### BACKGROUND

Over the years, various drugs have been developed to assist in the treatment of a wide variety of ailments and diseases. However, in many instances such drugs are not capable of being administered either orally or intravenously without the risk of various detrimental side effects.

CMV retinitis is a disease that is characterized by inflammation of the retina caused by infection with cytomegalovirus. CMV retinitis is one of the most common causes of sight-threatening infections among people with HIV. The symptoms include loss of visual acuity, blind spots, and the loss of peripheral vision. Left untreated, CMV retinitis can lead to blindness.

Intravenous ganciclovir (GCV) is effective in the treatment of CMV retinitis in AIDS patients, but bone marrow toxicity limits its usefulness. Continuous maintenance GCV therapy is necessary to prevent progression or recrudescence of the disease, but despite maintenance therapy a significant number of patients experience a relapse during treatment. Additionally, there are other risks and problems associated with systemic GCV administration.

Intravitreal GCV injections administered once or twice weekly have resulted in temporary remission of CMV retinitis in AIDS patients. Intravitreal GCV injections may provide a higher intraocular drug concentration than systemic therapy and reduce the incidence of neutropenia. However, current treatment of CMV retinitis in AIDS patients is clearly suboptimal. Ganciclovir is virustatic and thus disease inhibition requires maintenance drug administration.

A more detailed explanation of the use of intravenous GCV and intravitreal injections of GCV can be found in U.S. Patent No. 5,902,598, herein incorporated in its entirety by reference. A discussion of the difficulties associated with the systemic therapy of cyclosporine A in the treatment of uveitis can be found in U.S. Patent Nos. 5,773,019 and 6,001,386, herein incorporated in their entirety by reference.

Accordingly, there exists a strong need for the elimination of the undesirable physiological problems associated with GCV treatment of CMV retinitis, while maintaining the advantageous properties of this treatment. Although delivering the drug locally with injections may minimize the systemic toxicity of GCV, repeated injection is not a practical mode of administration.

Due to the risks that certain drugs impose, researchers have developed systems for administering such drugs to aid in the treatment of these ailments and diseases. A general discussion of drug delivery control systems is provided in Controlled Drug Delivery (Part I), Xue Shen Wu, Ph.D. pp32, 33, 44-46, 63, 66, and 67 (Technomic Publishing Co. Inc., 1996), the entire contents of which are incorporated herein by reference. The systems have been designed largely to reduce and to control the release rate of incorporated drugs. However, these systems fail to achieve the advantages claimed by the present invention.

For example, U.S. Patent No. 4,014,335 to Arnold, relates to various ocular inserts that act as a deposit or drug reservoir for slowly releasing a drug into the tear film for prolonged periods of time. These inserts are fabricated as a three-layer laminate of flexible polymeric materials that are biologically inert, non-allergenic, and insoluble in tear fluid. To initiate the therapeutic programs of these devices, the ocular inserts are placed in the cul-de-sac between the sclera of the eyeball and the eyelid for administering the drug to the eye. Multiple layer laminate systems can present a challenge to reproducibly manufacture and are more difficult to produce by large-scale or commercial manufacturing procedures.

The device of U.S. Patent No. 3,416,530 is manufactured with a plurality of capillary openings that communicate between the exterior of the device and the interior chamber generally defined from a polymeric membrane. While the capillary openings in this construction are effective for releasing certain drugs to the eye, they add considerable complexity to the manufacture of the device because it is difficult to control the size of these openings in commercial manufacturing using various polymers.

U.S. Patent No. 3,618,604 describes a device that does not involve such capillary openings, but instead provides for the release of the drug by diffusion through a polymeric membrane. The device, as disclosed in a preferred embodiment, comprises a sealed container with the drug contained in an interior chamber. Nonetheless, as described in U.S. Patent No. 4,014,335, certain problems have been identified with such devices such as the difficult task of sealing the margins of the membrane to form the container. In addition, stresses and strains introduced into the membrane walls from deformation during manufacturing of those devices may cause the reservoir to rupture and leak.

U.S. Patent No. 6,001,386 to Ashton, et al. relates to an implantable sustained release drug delivery device with an inner core containing an effective amount of a low solubility agent covered by a non-bioerodible polymer coating layer that is permeable to the low solubility agent disclosed. The above described systems and devices are intended to provide sustained release of drugs effective in treating patients at a desired local or systemic level for obtaining certain physiological or pharmacological effects. However, there are many disadvantages associated with their use, including the fact that it is often difficult to obtain the desired release rate of the drug.

The need for a better release system is especially significant in the treatment of CMV retinitis. Thus, there remains a long-felt need in the art for an improved device for providing sustained release of a drug to a patient to obtain a desired local or systemic physiological or pharmacological effect.

Prior to the development of the present invention, there was a drug delivery device developed that ameliorated many of the problems associated with sustained release drug delivery. The device, which is disclosed in U.S. Patent No. 5,378,475 (incorporated herein by reference in its entirety), included a first coating essentially impermeable to the passage of the effective agent and a second coating permeable to the passage of the effective agent. In the device, the first coating covered at least a portion of the inner core; however, at least a small portion of the inner core is not coated with the first coating layer. The second coating layer essentially completely covers the first coating layer and the uncoated portion of the inner core. The portion of the inner core which is not coated with the first coating layer allows passage of the agent into the second coating layer thus allowing controlled release.

While the devices described in U.S. Patent No. 5,378,475 solve many of the aforementioned problems pertaining to drug delivery, the devices and the method of making the devices are not without some problems. In particular, polymers suitable for coating the inner core are frequently relatively soft and technical difficulties can arise in production of uniform films. This is especially true when attempting to coat non-spherical bodies with edges (such as a cylindrical shape). In such cases, relatively thick films must be applied to achieve uninterrupted and uniform coatings, which adds significant bulk to the device. Thus, the devices tend to be larger than necessary as a result of the thickness needed to seal the ends of the inner core. In addition to adding bulk, multiple layer devices are more difficult to manufacture reproducibly and are more difficult to produce by commercial manufacturing procedures. Also, the various layers can be made of materials that are relatively incompatible with one another adding to the difficulties in coating. Often devices such as these require manual assembly that is time consuming, limits available supply, and adds variability.

U.S. Patent No. 5,902,598 also presents solutions to some of the problems associated with manufacturing small devices. The device in U.S. Patent No. 5,902,598 includes a third permeable coating layer that essentially completely covers the device. While the third coating layer improves the structural integrity of the device and helps to prevent potential leakage, some manufacturing difficulties can limit scaled up manufacturing. For example, consistent application of the outermost coating layer and reproducibility in manufacturing can be problems with designs which require manual assembly, a significant number of steps in the assembly process, or outer dip coatings.

In addition, depending on the materials selected for the outermost coating layer of the devices in U.S. Patent Nos. 5,902,598 and 5,378,475, there may exist a need to cure the entire device including the agent. Depending on the amount of curing required and the agents used, in some applications this could result in undesirable degradation of the active.

The problem of device size is extremely important in the design of devices for implantation into the limited anatomical spaces such as small organs like the eye. Larger devices require more complex surgery to both implant and remove. The increased complexity can result in complication, longer healing or recovery periods, and potential side effects (e.g. increased chance of astigmatism). Further, the extra polymer required to achieve a uniform coating reduces the potential internal volume of the implant and hence limits the amount of drug that can be delivered, potentially limiting both efficacy and duration.

Also, failure of some of these devices in use can lead to a dumping of the agent, which can cause harm to the mammalian organism being treated.

It would, therefore, be desirable to have a structurally stable device that can be reproducibly manufactured and manufactured by commercial techniques. As a result of all of the above, there remains a long felt need in the art for an improved device for providing sustained release of a drug to a mammalian organism to obtain a desired local or systemic physiological or pharmacological effect especially for ocular use.

### SUMMARY OF THE INVENTION

In accordance with the present invention there is provided a sustained release drug delivery device comprising:
a) a drug core comprising an inner core comprising at least one agent effective in obtaining a diagnostic effect or effective in obtaining a desired local or systemic physiological or pharmacological effect and a polymer coating layer, the polymer being permeable to the passage of said agent, wherein the polymer coating layer covers the inner core; and
b) a reservoir essentially impermeable to the passage of said agent that surrounds and defines an internal compartment to accept said drug core, said reservoir comprising at least one passageway, said passageway allowing passage of said agent out of the inner core, through the polymer coating layer, through the passageway, and out of the device.

The present invention is further directed to a method of manufacturing a sustained release drug delivery device comprising:
a) manufacturing an inner core comprising at least one agent effective in obtaining a diagnostic effect or effective in obtaining a desired local or systemic physiological or pharmacological effect;
b) forming a drug core by coating said inner core with a polymer coating layer permeable to the passage of said agent;
c) encapsulating said drug core in impermeable polymer essentially impermeable to the passage of said agent that surrounds and defines an internal compartment to accept said drug core, said unitary cup comprising an open top end with at least one lip extending around at least a portion of the said open top end of said unitary cup; and
d) making at least one passageway through said impermeable polymer layer allowing passage of said agent out of said inner core, through said polymer coating layer, and out of said passageway.

### BRIEF DESCRIPTION OF THE DRAWINGS

The drawings, which are not drawn to scale, are set forth to illustrate various embodiments of the invention. The drawings are as follows:

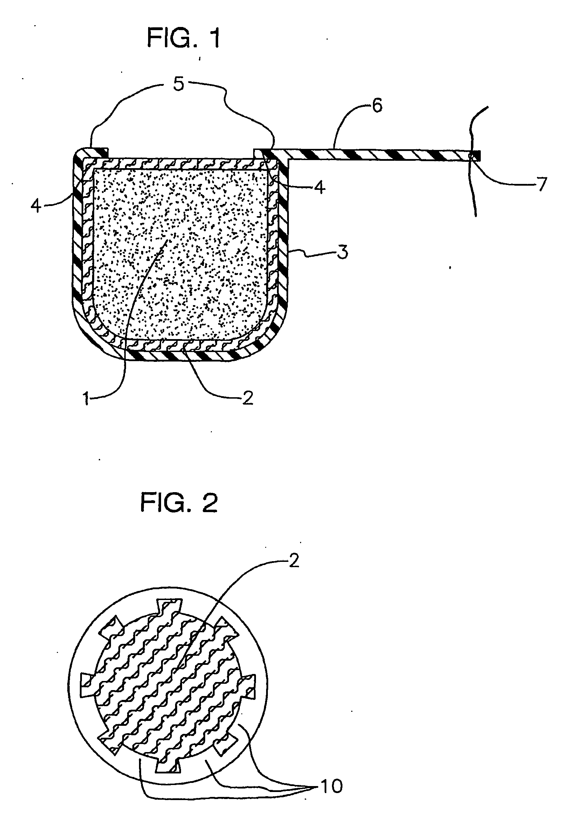

FIG. 1 of the present invention is an enlarged cross-sectional view down the center of one embodiment of the sustained release drug delivery device showing a unitary cup with a lip extending inward around some portion of the open end of the cup, a drug core formed of an inner core coated with a permeable polymer layer, the cup acting as a reservoir for the drug core.
FIG. 2 of the present invention is an enlarged top view of another embodiment of the sustained release drug delivery device showing a unitary cup with a plurality of lips extending inward around at least a portion of the open end of the cup and a drug core formed of an inner core coated with a permeable polymer layer.

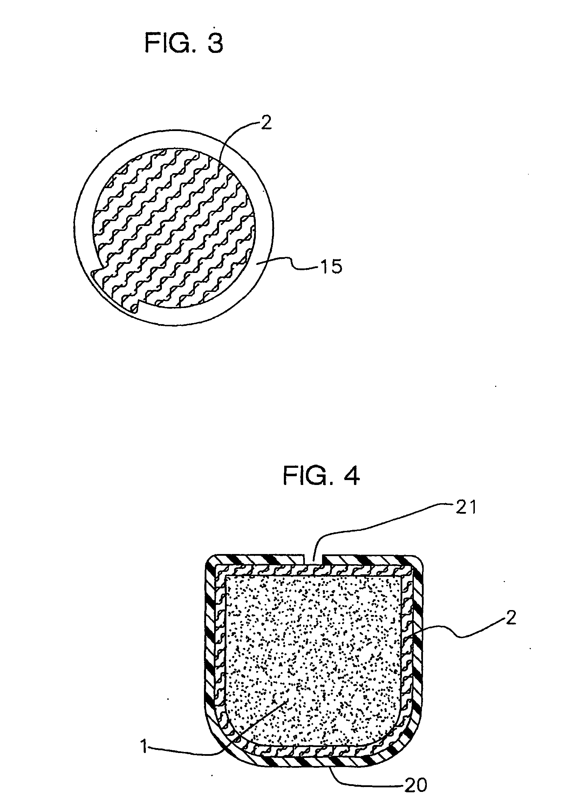

FIG. 3 of the present invention is an enlarged top view of the embodiment of the sustained release drug delivery device in FIG 1 showing a lip extending outward around only a portion of the open top end of the cup and a drug core formed of an inner core coated with a permeable polymer layer.
FIG. 4 of the present invention is an enlarged cross-sectional view through the center of the sustained release drug delivery device showing a unitary reservoir with a passageway and a drug core formed of an inner core coated with a permeable polymer layer.

### DETAILED DESCRIPTION OF THE INVENTION

The inventor has unexpectedly discovered a sustained release drug delivery device design that is structurally stabile, provides additional safety, and can be more easily and reproducibly manufactured than current designs that are known in the art.

In one preferred embodiment, the device includes an impermeable unitary cup made of silicone with an integral suture tab, the unitary cup acts as a reservoir for a drug core containing an agent such as fluocinolone acetonide. A hole through the proximal end of the suture tab enables a suture to be used for securing the device. The open end of the unitary cup has lips extending inwardly around a portion of the top open end of said cup. An inner core is formed of a pellet of fluocinolone acetonide. The drug core is formed by coating the inner core with a permeable polymer solution of 10% PVA. The PVA coating is allowed to dry. The drug core is then cured for 45 minutes at 135-145°C. The drug core is placed into the cup with lips which acts as a reservoir surrounding the coated inner core keeping it in place. ,

The expression "agent" as used herein broadly includes any compound, composition of matter, or mixture thereof that can be delivered from the device to produce a beneficial and useful result.

The term "impermeable" refers to a material that is sufficiently impermeable to environmental fluids as well as ingredients contained within the delivery device, such that the migration of such fluids and ingredients into or out of the device through the impermeable material is so low as to have substantially no adverse impact on the function of the device.

The term "permeable" refers to a material that is capable of being passed through or permeated. Permeating includes passing through openings, holes, pores, or intersections. The term "inner core" refers to any drug supply, drug depot, drug in suspension, reservoir or drug matrix. It includes one or more agents necessary to obtain the desired diagnostic effect or local or systemic physiological or pharmacological effect. It includes any excipients, suspending agents, or binders. Reference may be made to any standard pharmaceutical textbook such as Remington's Pharmaceutical Sciences. The inner core can be in liquid form, solid form, in dispersion, or any other form known in the art. Solid dose includes all conventional solid dose forms known in the art including tablets and pellets. Dispersions include all conventional forms known in the art, such as liquid in liquid dispersions and solid in liquid dispersions.

The expression "passageway" as used herein comprises means and methods suitable for releasing the agent from the device. The expression includes an aperture, orifice, or bore through the device. The passageway can be formed by mechanical procedures such as erosion, laser, or molding; and chemical procedures.

Referring to the drawing figures, like reference numerals designate identical or corresponding elements throughout the several figures.

Turning now to the drawings in detail, which examples are not to be construed as limiting, one embodiment of a device is indicated in Figure 1. While the device shown in Figure 1 is generally U like in shape, the cup can be any open container or bowl of any shape. Figure 1 is a cross sectional view of a drug delivery device in accordance with the present invention. Figure 1 includes an impermeable unitary cup 3, the cup 3 has lips 4 extending inward around the open top end 5 of the cup 3; the cup 3 contains an inner core 1 coated with a polymer layer 2 that is permeable to the passage of the agent contained in the inner core 1. Together the inner core 1 and the polymer layer 2 form the drug core. The drug core is positioned below the lips 4 such that the lips 4 interact with the drug core holding it in position and closing the open top end 5 of the cup 3. The lips 4 are the same impermeable material as the unitary cup 3 and protrude inwardly from the top open end 5 of the cup 3. The cup 3 and lips 4 are formed in a single unitary design to provide structural integrity to the device and facilitate manufacturing and handling. The lips 4 are designed to hold the drug core in place during use. They can vary in size or shape. The lips 4 of the present invention include nubs, tabs, ridges, and any other raised or protruding member.

The agent diffuses out of the inner core 1, through the polymer coating 2, and out the open top end 5 of the unitary cup 3. Glue, polymers, or other adhesion means can be employed to further bond the drug core to the cup.

The cup 3 further comprises an integral suture tab 6 with a hole 7 through the proximal end through which a suture can be placed to anchor the device to a structure of the organism requiring treatment. The proximal end of the suture tab is at the point of attachment, i.e. the point where the suture is attached. The preferred point of attachment is at the end of the suture tab opposite the cup.

The location of the suture and the structure the device is sutured to can be any that meet with current surgical techniques known in the art, such as the sclera of the eye. Depending upon the location of administration, the devices of the current invention may not require suturing in position.

Making the cup and suture tab in a single unitary design provides structural integrity to the device, and facilitates manufacturing and handling as one integral structure. In addition, by eliminating the assembly step of attaching the suture tab onto the cup, the single unitary design decreases variability in the size and shape of the device.

Providing a suture hole 7 at the proximal end of the suture tab of the device enables the surgeon to attach the device without additional steps. Some materials, such as cured polyvinyl alcohol (PVA), are also very difficult to create a suture hole in once the device is assembled without causing cracks or breaks in the suture tab.

The invention further relates to a method for treating a mammalian organism to obtain a desired local or systemic physiological or pharmacological effect. The method includes administering the sustained release drug delivery device to the mammalian organism and allowing the agent effective in obtaining the desired local or systemic physiological or pharmacological effect to pass out of the inner core, through the polymer layer, and out the open top end of the unitary cup. The term "administering", as used herein, means positioning, inserting, injecting, implanting, or any other means for exposing the device to a mammalian organism. The route of administration depends on a variety of factors including type of response or treatment, type of agent, and the preferred site of administration. However, the preferred method is to insert the device into the target organ. In ocular applications, more preferably through a surgical procedure followed by suturing the device in place.

Figure 2 illustrates an enlarged top view of another exemplary embodiment of a sustained release drug delivery device of the present invention. The view in Figure 2 is the top of a unitary cup comprising a plurality of lips 10 extending inwardly around the open top end of the cup. The drug core 11 is comprised of an inner core comprising an agent and a polymer layer coating the inner core. The drug core 11 is held in place by the lips 10 extending inwardly around the top open end of the cup.

Figure 3 is an enlarged top view of another exemplary embodiment of a sustained release drug delivery device of the present invention. The view in Figure 5 is the top of a unitary cup comprising a single lip 15. The drug core 11 is comprised of an inner core comprising an agent and a polymer layer coating the inner core. The drug core 11 is held in place by the lip 15 extending inwardly around the top open end of the cup. The single lip can extend around the entire diameter of the top open end of the cup or extend around some portion, as illustrated in Figure 3.

Figure 4 is an enlarged cross-sectional view down the center of a sustained release drug delivery device of the present invention. Figure 4 includes an impermeable reservoir 20, the reservoir 20 having a passageway 21 in the center of one wall. The reservoir contains a drug core which is comprised of an inner core 1 comprising an agent and a polymer layer 2 which coats the inner core.

In combination with the examples above, a variety of methods may also be utilized to provide adhesion of the polymer coated inner core to the unitary cup portion of the device. These methods include the use of adhesives, polymers such as PVA, or any other procedure known in the art to provide adhesion at the points of contact between the permeable plug and the unitary cup. The methods to improve adhesion will vary depending on the materials that the components are manufactured from.

For example, the polymer coated inner cores or the unitary cups of the present invention may also be treated before or after assembly with an adhesive, which would serve to further secure the drug core in the device. The sealant must be permeable to the agent or agents in the device. For example, a few drops of a permeable polymer could be placed in the unitary cup before inserting the polymer coated inner core into the unitary cup device.

The inner core of the present invention is coated with a polymer permeable to the passage of the agent(s) contained in the inner core. The inner core is coated to provide ease of assembly. The present design is easier to assemble then current designs known in the art and can be more easily made by commercial manufacturing techniques. Some of the coating layer may be scrapped off or removed during assembly as long as the exposed portion of the drug core is adequately coated.

The inner core contains an agent effective in obtaining a desired local or systemic physiological or pharmacological effect. The following classes of agents could be incorporated into the devices of the present invention: anesthetics and pain killing agents such as lidocaine and related compounds and benzodiazepam and related compounds; anti-cancer agents such as 5-fluorouracil, adriamycin and related compounds; anti-fungal agents such as fluconazole and related compounds; anti-viral agents such as trisodium phosphomonoformate, trifluorothymidine, acyclovir, ganciclovir, DDI and AZT; cell transport/mobility impending agents such as colchicine, vincristine, cytochalasin B and related compounds; antiglaucoma drugs such as beta-blockers: timolol, betaxolol, atenalol, etc; antihypertensives; decongestants such as phenylephrine, naphazoline, and tetrahydrazoline; immunological response modifiers such as muramyl dipeptide and related compounds; peptides and proteins such as cyclosporin, insulin, growth hormones, insulin related growth factor, heat shock proteins and related compounds; steroidal compounds such as dexamethasone, prednisolone and related compounds; low solubility steroids such as fluocinolone acetonide and related compounds; carbonic anhydrize inhibitors; diagnostic agents; antiapoptosis agents; gene therapy agents; sequestering agents; reductants such as glutathione; antipermeability agents; antisense compounds; antiproliferative agents; antibody conjugates; antidepressants; bloodflow enhancers; antiasthmatic drugs; antiparasiticagents; non-steroidal anti inflammatory agents such as ibuprofen; nutrients and vitamins: enzyme inhibitors: antioxidants; anticataract drugs; aldose reductase inhibitors; cytoprotectants; cytokines, cytokine inhibitors, and cytokin protectants; uv blockers; mast cell stabilizers; and anti neovascular agents such as antiangiogenic agents like matrix metalloprotease inhibitors.

Examples of such agents also include neuroprotectants such as nimodipine and related compounds; antibiotics such as tetracycline, chlortetracycline, bacitracin, neomycin, polymyxin, gramicidin, oxytetracycline, chloramphenicol, gentamycin, and erythromycin; antiinfectives; antibacterials such as sulfonamides, sulfacetamide, sulfamethizole, sulfisoxazole; nitrofurazone, and sodium propionate; antiallergenics such as antazoline, methapyriline, chlorpheniramine, pyrilamine and prophenpyridamine; antiinflammatories such as hydrocortisone, hydrocortisone acetate, dexamethasone 21-phosphate, fluocinolone, medrysone, methyiprednisolone, prednisolone 21-phosphate, prednisolone acetate, fluoromethalone, betamethasone and triminolone; miotics and anticholinesterase such as pilocarpine, eserine salicylate, carbachol, di-isopropyl fluorophosphate, phospholine iodine, and demecarium bromide; mydriatics such as atropine sulfate, cyclopentolate, homatropine, scopolamine, tropicamide, eucatropine, and hydroxyamphetamine; svmpathomimetics such as epinephrine; and prodrugs such as those described in Design of Prodrugs, edited by Hans Bundgaard, Elsevier Scientific Publishing Co., Amsterdam, 1985. In addition to the above agents, other agents suitable for treating, managing, or diagnosing conditions in a mammalian organism may be placed in the inner core and administered using the sustained release drug delivery devices of the current invention. Once again, reference may be made to any standard pharmaceutical textbook such as Remington's Pharmaceutical Sciences for the identity of other agents.

Any pharmaceutically acceptable form of such a compound may be employed in the practice of the present invention, i.e., the free base or a pharmaceutically acceptable salt or ester thereof. Pharmaceutically acceptable salts, for instance, include sulfate, lactate, acetate, stearate, hydrochloride, tartrate, maleate and the like.

A large number of polymers can be used to construct the devices of the present invention. The only requirements are that they are inert; non-immunogenic and of the desired permeability. Materials that may be suitable for fabricating the device include naturally occurring or synthetic materials that are biologically compatible with body fluids and body tissues, and essentially insoluble in the body fluids with which the material will come in contact. The use of rapidly dissolving materials or materials highly soluble in body fluids are to be avoided since dissolution of the wall would affect the constancy of the drug release, as well as the capability of the device to remain in place for a prolonged period of time.

Naturally occurring or synthetic materials that are biologically compatible with body fluids and eye tissues and essentially insoluble in body fluids which the material will come in contact include, but are not limited to, glass, metal, ceramics, polyvinyl acetate, cross-linked polyvinyl alcohol, cross-linked polyvinyl butyrate, ethylene ethylacrylate copolymer, polyethyl hexylacrylate, polyvinyl chloride, polyvinyl acetals, plasiticized ethylene vinylacetate copolymer, polyvinyl alcohol, polyvinyl acetate, ethylene vinylchloride copolymer, polyvinyl esters, polyvinylbutyrate, polyvinylformal, polyamides, polymethylmethacrylate, polybutylmethacrylate, plasticized polyvinyl chloride, plasticized nylon, plasticized soft nylon, plasticized polyethylene terephthalate, natural rubber, polyisoprene, polyisobutylene, polybutadiene, polyethylene, polytetrafluoroethylene, polyvinylidene chloride, polyacrylonitrile, cross-linked polyvinylpyrrolidone, polytrifluorochloroethylene, chlorinated polyethylene, poly(1 ,4'-isopropylidene diphenylene carbonate), vinylidene chloride, acrylonitrile copolymer, vinyl chloride-diethyl fumerale copolymer, butadiene/styrene copolymers, silicone rubbers, especially the medical grade polydimethylsiloxanes, ethylene-propylene rubber, silicone-carbonate copolymers, vinylidene chloride-vinyl chloride copolymer, vinyl chloride-acrylonitrile copolymer and vinylidene chloride-acrylonitride copolymer.

The device can be formulated in any convenient shape. For example, the device can be of any geometric shape dimensionally suitable for insertion in the eye. Thus, the device can be ellipsoid, rectangular, round, etc.

The dimensions of the device can vary with the size of the device, the size of the core or reservoir, and the membrane that surrounds the core or reservoir. The physical size of the device should be selected so that it does not interfere with physiological functions at the implantation site of the mammalian organism. The targeted disease state, type of mammalian organism, location of administration, and agents or agent administered are among the factors which would effect the desired size of the sustained release drug delivery device.

The devices according to the present invention may be made in a variety of ways. For example, if the unitary cup or reservoir is going to be made entirely of polymer, then the polymer can be injection molded or die cast into a desired shape and size. The inner core can be made as any solid dose form such as a tablet or pellet. The drug core can then be formed by coating the solid inner core with permeable polymer using any coating means currently known in the art. The drug core could also be formed with an inner core that is a drug in liquid form or suspension that is encapsulated in a permeable polymer.

The reservoir can be made in one piece, such as by encapsulating the polymer coated inner core then boring out the desired passageway(s). The size or number of passageways can be selected to achieve the desired release rate. The reservoir can also be formed using a unitary cup and inserting a plug of impermeable material. The assembled device having at least one passageway. The unitary cup can have lip(s) or groove(s) around the open top end which interact with the impermeable plug holding it in place and closing the open top end of the cup. Due to the elastic nature of some polymers, such as silicone, the same result could also be achieved by essentially molding the reservoir as one piece and stretching the passageway wide enough to insert the drug core through the. passageway.

The preceding descriptions of how to make the devices of the present invention is merely illustrative and should not be considered as limiting the scope of the invention in any way. In particular, the methods of making the device depend on the identity of the agent.

The devices may be surgically implanted at or near the site of action. This is the case for devices of the present invention used in treatment of ocular conditions, primary tumors, rheumatic and arthritic conditions, and chronic pain. The devices may also be implanted subcutaneously, intramusclarly, intraarterially, or intraperitoneally. This is the case when devices are to give sustained systematic levels and avoid premature metabolism. In addition, such devices may be administered orally.

Once in place, the device functions as a drug reservoir gradually releasing drug to the organ such as the eye and surrounding tissue. This device is particularly useful for treating ocular conditions such as glaucoma, proliferative vitreoretimopathy, diabetic retinopathy, uveitis, and keratitis. The device is also particularly useful as an ocular device in treating mammalian organisms suffering from cytomegalovirus retinitis wherein the device is surgically implanted within the vitreous of the eye.

As would be readily understood by one skilled in the art, the preferred amounts, materials, and dimensions depend on the method of administration, the effective agent used, the polymers used, the desired release rate and the like. Likewise, actual release rates and release duration depend on a variety of factors in addition to the above, such as the disease state being treated, the age and condition of the patient, the route of administration; as well as other factors which would be readily apparent to those skilled in the art. All of the forgoing U.S. Patents and other publications are expressly incorporated by reference herein in each of their entities.

Thus, the devices of the present invention provide many important advantages over previously known sustained release drug delivery devices. The unitary cup and polymer coated inner core design of the present invention provide an improved device that maintains its physical and chemical integrity in both the environments of use and in the presence of agent during the controlled and continuous dispensing of agent over a prolonged period of time.

Due to the structural integrity of the present design, the need for multiple layers can be eliminated. The ease of making the devices of the present invention minimizes stresses, strains, and deformations of the devices during manufacture which may cause the reservoir to rupture and leak. The leaking of agent can result in harm to the patient and is a significant concern in the manufacture of implantable devices.

In the unlikely event of a device failure, the polymer coated drug core will provide additional security that an undesired "dumping" of the agent will not occur. This is especially important when organs such as the eye are being treated.

The unitary cup or reservoir and the drug core design in the present invention results in a device that is more easily and reproducibly manufactured then current designs known in the art. Manufacturing with the single unitary cup or reservoir and drug core minimizes the number of steps and decreases potential variability in assembly. The present design also allows for mechanized manufacture. Eliminating manual assembly greatly decreases the potential variability in the finished product.

Another advantage of the devices of the present invention is the ease of construction by more standard manufacturing techniques into devices with different release rates. The number and size of the passageways in the reservoir embodiment of the present invention can be used to control diffusion properties to achieve a desired release rate. Varying the composition of the drug core can also be used to achieve a desired release rate. The same reservoir or unitary cup can be used for implants with different release rates making it possible to use a single manufacturing line or type of equipment.

In addition, the use of a single unitary cup and drug core eliminates the difficulties of sealing the margins faced by other devices in the prior art. This permits the therapeutic program to be precisely controlled and the release of drug to be constant and predicted with accuracy.

The following specific examples demonstrate some of the sustained release drug delivery device designs of the present invention. However, it is to be understood that these examples are for illustrative purposes only and do not purport to be wholly definitive as to the conditions and scope.

### EXAMPLE 1

A device according to the present invention is prepared. The unitary cup is made of silicone and has eight inwardly extending lips around the top open end of the cup. The unitary cup has an integral suture tab with a hole at the end of the tab opposite the cup. An inner core is formed as a pellet composed of a 2.5 mg of fluocinolone acetonide. The inner core is coated with a 10% solution of PVA. The PVA coated inner core is cured for 50 minutes at 135-145° C. The polymer coated inner core is then inserted into the unitary cup. The lips of the unitary cup act to hold the permeable plug in place.

### EXAMPLE 2

The device of example 1 above is placed in a vial with 2.0 mL of a release media of 0.1 Sodium Acetate, pH 4.2. The vial is maintained in a 37°C bath for 24 hours. After 24 hours, the vial is inverted to ensure homogeneity and the device is removed to a new vial with fresh media. This process is repeated for each day. The media is tested to determine the amount of the drug and verifies that it is being released from the device. From the data that is collected, the release rate of the device can be determined.

From the foregoing description, one of ordinary skill in the art can easily ascertain the essential characteristics of the instant invention, and without departing from the spirit and scope thereof, can make various changes and/or modifications of the inventions to adapt it to various usages and conditions. As such, these changes and/or modifications are properly, equitably, and intended to be, within the full range of equivalence of the following claims.

## Claims

1. A sustained release drug delivery device comprising:
a) a drug core comprising an inner core comprising at least one agent effective in obtaining a diagnostic effect or effective in obtaining a desired local or systemic physiological or pharmacological effect and a polymer coating layer, the polymer being permeable to the passage of said agent, wherein the polymer coating layer covers the inner core; and
b) a reservoir essentially impermeable to the passage of said agent that surrounds and defines an internal compartment to accept said drug core, said reservoir comprising at least one passageway, said passageway allowing passage of said agent out of the inner core, through the polymer coating layer, through the passageway, and out of the device.

2. The sustained release drug delivery device according to claim 1, wherein said inner core comprises a plurality of agents.

3. The sustained release drug delivery device according to claim 1 or claim 2, wherein said inner core comprises an effective amount of a low solubility agent.

4. The sustained release drug delivery device according to any preceding claim, wherein said agent is selected from a group consisting of immune response modifiers, neuroprotectants, corticosteroids, angiostatic steroids, anti-parasitic agents, anti-glaucoma agents, anti-biotics, anti-sense compounds, anti-angiogentic compounds, differentiation modulators, anti-viral agents, anti-cancer agents, and nonsteroidal anti-inflammatory agents.

5. The sustained release drug delivery device according to any preceding claim, wherein said reservoir is made of a polymer or a metal.

6. The sustained release drug delivery device according to any preceding claim, wherein said reservoir further comprises an integral suture tab.

7. The sustained release drug delivery device according to claim 6, wherein said reservoir is made of silicone.

8. The sustained release drug delivery device according to claim 7, wherein said polymer coating layer is made of PVA.

9. The sustained release drug delivery device according to claim 6, wherein said suture tab has a hole through the proximal end through which a suture can be placed to anchor the device to a structure.

10. The sustained release drug delivery device according to claim 1, wherein said reservoir further comprises:
a) a unitary cup essentially impermeable to the passage of said agent that surrounds and defines an internal compartment to accept said drug core, said unitary cup comprising an open top end with at lease one recessed groove around at least some portion of said open top end of said unitary cup; and
b) a plug which is essentially impermeable to the passage of said agent, said plug is positioned at said open top end of said unitary cup wherein said groove interacts with said plug holding it in position and closing said open top end.

11. The sustained release drug delivery device according to claim 1, wherein said reservoir further comprises:
a) a unitary cup essentially impermeable to the passage of said agent that surrounds and defines an internal compartment to accept said drug core, said unitary cup comprising an open top end and at least one lip around at lease a portion of said open top end of said unitary cup; and
b) a plug which is essentially impermeable to the passage of said agent, said plug is positioned at said open top end of said unitary cup wherein said lip interacts with said plug holding it in position and closing said open top end.

12. A method of manufacturing a sustained release drug delivery device comprising:
a) manufacturing an inner core comprising at least one agent effective in obtaining a diagnostic effect or effective in obtaining a desired local or systemic physiological or pharmacological effect;
b) forming a drug core by coating said inner core with a polymer coating layer permeable to the passage of said agent;
c) encapsulating said drug core in impermeable polymer essentially impermeable to the passage of said agent that surrounds and defines an internal compartment to accept said drug core, said unitary cup comprising an open top end with at least one lip extending around at least a portion of the said open top end of said unitary cup; and
d) making at least one passageway through said impermeable polymer layer allowing passage of said agent out of said inner core, through said polymer coating layer, and out of said passageway.

13. The method of manufacturing a sustained release drug delivery device according to claim 12, comprising the further step of curing said drug core prior to insertion into said unitary cup.
